# EUROPEAN PATENT SPECIFICATION

(11) **EP 2 049 673 B1**
(45) Date of publication and mention of the grant of the patent: **13.04.2011**
(21) Application number: 07836542.6
(22) Date of filing: 03.08.2007
(51) Int. Cl.: C12P 7/06, C12P 19/14

(54) **NATIVE GRAIN AMYLASES IN ENZYME COMBINATIONS FOR GRANULAR STARCH HYDROLYSIS**
NATIVE KORNAMYLASEN IN ENZYMKOMBINATIONEN ZUR HYDROLYSE GRANULARER STÄRKE
AMYLASES DE CÉRÉALES NATURELLES DANS DES ASSOCIATIONS D'ENZYMES POUR L'HYDROLYSE D'AMIDON GRANULÉ

(30) Priority: 11.08.2006 US 837366 P
(43) Date of publication of application: 22.04.2009
(73) Proprietor: Danisco US Inc., Palo Alto, CA 94304 (US)
(72) Inventor: KUMAR, Manoj, Fremont, CA 94555 (US)
(74) Representative: Kiddle, Simon John
(86) International application number: PCT/US2007/017485
(87) International publication number: WO 2008/021050

(56) References cited:
- WO-A-2004/081193
- WO-A-2005/069849
- DATABASE BIOSIS [Online] BIOSCIENCES INFORMATION SERVICE, PHILADELPHIA, PA, US; April 1998 (1998-04), LUO JINXIAN ET AL: "Expression and secretion of barley alpha-amylase and A. niger glucoamylase in Saccharomyces cerevisiae" XP002464125 Database accession no. PREV199800301619 & SCIENCE IN CHINA SERIES C LIFE SCIENCES, vol. 41, no. 2, April 1998 (1998-04), pages 113-118, ISSN: 1006-9305
- DATABASE BIOSIS [Online] BIOSCIENCES INFORMATION SERVICE, PHILADELPHIA, PA, US; March 2006 (2006-03), WONG DOMINIC ET AL: "Synergistic action of barley alpha-amylase and Lentinula edodes glucoamylase on raw starch hydrolysis" XP002464126 Database accession no. PREV200600342722 & FASEB JOURNAL, vol. 20, no. 4, Part 1, March 2006 (2006-03), page A42, EXPERIMENTAL BIOLOGY 2006 MEETING; SAN FRANCISCO, CA, USA; APRIL 01 05, 2006 ISSN: 0892-6638 -& WONG D W S ET AL: "Synergistic action of recombinant alpha-amylase and glucoamylase on the hydrolysis of starch granules" PROTEIN JOURNAL, vol. 26, no. 3, April 2007 (2007-04), pages 159-164, XP002464124 ISSN: 1572-3887
- KUMAGAI M H ET AL: "CONVERSION OF STARCH TO ETHANOL IN A RECOMBINANT SACCHAROMYCES CEREVISIAE STRAIN EXPRESSING RICE ALPHA-AMYLASE FROM A NOVEL PICHIA PASTORIS ALCOHOL OXIDASE PROMOTER" BIO/TECHNOLOGY, NATURE PUBLISHING CO. NEW YORK, US, vol. 11, May 1993 (1993-05), pages 606-610, XP002037995 ISSN: 0733-222X
- LIAKOPOULOU-KYRIAKIDES MARIA ET AL: "Synergistic hydrolysis of crude corn starch by alpha-amylases and glucoamylases of various origins" CEREAL CHEMISTRY, AACC INTERNATIONAL, ST PAUL, MN, US, vol. 78, no. 5, September 2001 (2001-09), pages 603-607, XP008083292 ISSN: 0009-0352
- MAEDA I ET AL: "DIGESTION OF BARLEZ STARCH GRANULES BY THE COMBINED ACTION OF ALPHA-AND BETA-AMYLASES PURIFIED FROM BARLEY AND BARLEY MALT" AGRICULTURAL AND BIOLOGICAL CHEMISTRY, JAPAN SOC. FOR BIOSCIENCE, BIOTECHNOLOGY AND AGROCHEM, TOKYO, JP, vol. 42, no. 2, 1978, pages 259-267, XP002042339 ISSN: 0002-1369
- JUGE ET AL: "The activity of barley alpha-amylase on starch granules is enhanced by fusion of a starch binding domain from Aspergillus niger glucoamylase" BIOCHIMICA ET BIOPHYSICA ACTA (BBA) - PROTEINS & PROTEOMICS, ELSEVIER, vol. 1764, no. 2, February 2006 (2006-02), pages 275-284, XP005309058 ISSN: 1570-9639

## Description

### CROSS-REFERENCE TO RELATED APPLICATIONS

This application claims priority to United States provisional application US 60/837,366, filed August 11, 2006,

### FIELD OF THE INVENTION

The present invention relates to starch hydrolyzing processes for obtaining fermentable sugars from starch in milled plant material at temperatures below the starch gelatinization temperature and further to the fermentation of the sugars to produce end products, such as ethanol.

### BACKGROUND OF THE INVENTION

Starch is an important raw material used extensively for industrial purposes such as the production of high fructose syrups and ethanol. Starch forms the major storage carbohydrate component in plant seeds and starch granules are generally resistant to degradation. Traditionally, high temperature and/or combinations of enzymes have been used to hydrolyze starch. Starch hydrolyzing enzymes such as alpha-amylases (E.C. 3.2.1.1.) which are endo-acting hydrolytic enzymes that hydrolyze internal alpha-1, 4 linkages of starch molecules are most frequently used in the industry. Alpha-amylases are produced by plants, animals and microorganisms. Starch hydrolysis from plant seeds and particularly starch hydrolysis from grains with alpha-amylase enzymes derived from microbial sources is routine in many industrial process wherein the first step of starch degradation is the hydrolysis of starch to glucose or the hydrolysis of starch to maltodextrins. Traditionally, these processes have been conducted at high temperatures, (e.g., temperatures higher than the initial gelatinization temperature of the starch comprising the substrate) (Corn: Chemistry and Technology, Watson S.A. et al. Eds., (1991) American Association of Cereal Chemists, Inc. and The Alcohol Textbook, 3rd Edition, Jacques et al. Eds., (1999), Nottingham University Press). More recently, the industry has explored the production of starch hydrolysis and alcohol production by fermentation using lower energy processes (e.g., at temperatures below the initial gelatinization temperature of the starch contained in the plant material) (USP 4,514,496, WO 03/066826; WO 04/081193; WO 04/106533; WO 04/080923 and WO 05/069840).

### SUMMARY OF THE INVENTION

The present invention relates to a process of hydrolyzing granular starch from milled plant material comprising contacting milled plant material with a combination of an exogenous plant alpha-amylase, a Trichoderma or Humicola glucoamylase and optionally microbial alpha-amylases at temperatures below the initial gelatinization temperature of the granular starch in the milled plant material to obtain fermentable sugars. The invention further relates to fermenting the fermentable sugars to end products in the presence of fermenting microorganisms.

In one aspect, the invention includes a process of hydrolyzing starch from milled plant material comprising granular starch, by contacting milled plant material with an exogenous plant alpha-amylase, and a glucoamylase at a temperature below the starch gelatinization temperature of the granular starch to produce oligosaccharides, and producing fermentable sugars. The process may also include fermenting the fermentable sugars in the presence of fermenting microorganisms at a temperature of between 10°C and 40°C for a period of time of 10 hours to 250 hours to produce alcohol. The milled plant material can be a slurry having granular starch, the slurry having a DS of between 5 and 60%, or alternatively a DS of between 25 and 40% or alternatively, a DS of between 15 and 45%. The alpha amylase can be added at between 0.001 and 30 AAU/DS.

In another aspect, the contacting step also includes contacting the milled plant material with a microbial alpha-amylase. The temperature can be between 50° and 70°C. The plant alpha-amylase can be an alpha-amylase from plants including: barley, wheat, rice corn, rye, sorghum, rice, millet, triticale, cassava, potato, sweet potato, sugar beet, sugarcane, soybean and pea. Preferably, the plant material is corn, milo, barley, wheat, rice or combinations thereof. In some important aspects, the plant is fractionated corn. In some aspects, the alcohol is ethanol and the method further includes recovering the ethanol.

In other aspects, the invention includes methods for producing ethanol by, contacting a slurry comprising granular starch obtained from plant material with an exogenous plant alpha-amylase capable of solubilizing granular starch at a temperature below the starch gelatinization temperature of the granular starch for a period of 5 minutes to 24 hours and a Trichoderma or Humicola glucoamylase, obtaining a substrate, and fermenting the substrate in the presence of a fermenting microorganism at a temperature of between 10°C and 40°C for a period of 10 hours to 250 hours to produce ethanol. In some aspects, the method also includes recovering the ethanol. The alpha-amylase can be an alpha-amylase such as those including: barley, wheat, rice corn, rye, sorghum, rice, millet, triticale, cassava, potato, sweet potato, sugar beet, sugarcane, soybean and pea. In some aspects, the method includes contacting the slurry with a microbial alpha amylase. The contacting step can be conducted at a temperature of between 50°C and 70°C. The method can also include clarifying the substrate before the fermenting step. In some aspects, the method can include adding additional enzymes to the contacting step, such as glucoamylases, phytases, proteases, cellulases and/or hemicellulases. In some preferred aspects, the additional enzyme is a phytase and/or a protease. In some aspects, the slurry has between 5 - 60% DS granular starch or alternatively between 20 - 40% DS. In some aspects, the method includes contacting the substrate with an aqueous solution comprising backset to dilute the %DS prior to the fermentation step. The granular starch can be obtained from corn, milo, barley, wheat, rice or combinations thereof.

### BRIEF DESCRIPTION OF THE DRAWINGS

FIG. 1 illustrates the production of ethanol (% v/v) from barley flour (32% DS) over fermentation time (hrs) under various treatments including: STARGEN 001 (-◊-); Barley AA + AnGA (-■-); Barley AA + TrGA (-●-) and Barley AA + HGA (-▲-).

FIG. 2 illustrates the production of ethanol (% v/v) from barley flour (32% DS) over fermentation time (hrs) under various treatments including: STARGEN 001(-◊-); HGA (-□-); Barley AA+STARGEN 001 (-∗-); Barley AA + HGA (-■-); and control (no enzyme added) (-●-).

### DETAILED DESCRIPTION OF THE INVENTION

Unless defined otherwise herein, all technical and scientific terms used herein have the same meaning as commonly understood by one of ordinary skill in the art to which this invention belongs.

Although any methods and materials similar or equivalent to those described herein can be used in the practice or testing of the present invention, the preferred methods and materials are described.

The invention will now be described in detail by way of reference only using the following definitions and examples.

### Definitions:

As used herein the term "starch" refers to any material comprised of the complex polysaccharide carbohydrates of plants, comprised of amylose and amylopectin with the formula (C₆H₁₀O₅)ₓ, wherein x can be any number.

The term "granular starch" refers to raw starch, that is starch in its natural form found in plant material (e.g. grains and tubers).

The term "dry solids content (ds)" refers to the total solids of a slurry in % on a dry weight basis. The term "slurry" refers to an aqueous mixture containing insoluble solids.

The term "fermentable sugars" refers to oligosaccharides and monosaccharides that can be converted into end products by fermentation with a fermenting microorganism.

The term "dextrins" refers to short chain polymers of glucose (*e.g*. 2 to 10 units).

The term "oligosaccharides" refers to any compound having 2 to 10 monosaccharide units joined in glycosidic linkages. These short chain polymers of simple sugars include dextrins.

The term "alpha-amylase (*e.g*., E.C. class 3.2.1.1)" refers to enzymes that catalyze the hydrolysis of alpha-1, 4-glucosidic linkages.

The terms "saccharifying enzyme" and "starch hydrolyzing enzymes" refer to any enzyme that is capable of converting starch to mono- or oligosaccharides (*e.g*. a hexose or pentose).

The terms "granular starch hydrolyzing (GSH) enzyme" and "enzymes having granular starch hydrolyzing (GSH) activity" refer to enzymes, which have the ability to hydrolyze starch in granular form.

The term "hydrolysis of starch" refers to the cleavage of glucosidic bonds with the addition of water molecules.

The term "endogenous plant alpha-amylase" means an enzyme having alpha-amylase activity that is expressed and produced by the plant material. As used herein the endogenous plant alpha-amylase may be heterologous or homologous.

The term "exogenous plant alpha-amylase" means an enzyme having alpha-amylase activity, which is derived from a plant alpha-amylase and is provided independently from the plant material that is used as a substrate for starch hydrolysis.

The term "microbial alpha-amylases" refers to enzymes having alpha-amylase activity which are derived from microbial sources (*e.g*. bacterial or fungal) and includes modified enzymes, active fragments and hybrids thereof

The term "heterologous" with reference to a polynucleotide or polypeptide refers to a polynucleotide or polypeptide that does not naturally occur in a host cell. It is intended that the term encompass proteins that are encoded by naturally occurring genes, mutated genes, synthetic genes and/or over-expressed genes.

The term "homologous" with reference to a polynucleotide or protein refers to a polynucleotide or protein that occurs naturally in the host cell.

The term "glucoamylase" refers to the amyloglucosidase class of enzymes (e.g., E.C.3.2.1.3, glucoamylase, 1, 4-alpha-D-glucan glucohydrolase). These are exo-acting enzymes, which release glucosyl residues from the non-reducing ends of amylose and amylopectin molecules.

The term "milled" is used herein to refer to plant material that has been reduced in size, such as by grinding, crushing, fractionating or any other means of particle size reduction.

The term "gelatinization" means solubilization of a starch molecule, generally by cooking, to form a viscous suspension.

The term "gelatinization temperature" refers to the lowest temperature at which gelatinization of a starch containing substrate begins. The exact temperature of gelatinization depends on the specific starch and may vary depending on factors such as plant species and environmental and growth conditions. The initial starch gelatinization temperature ranges for a number of granular starches which may be used in accordance with the processes herein include barley (52°C to 59°C), wheat (58°C to 64°C), rye (57°C to 70°C), corn (62°C to 72°C), high amylose corn (67°C to 80°C), rice (68°C to 77°C), sorghum (68°C to 77°C), potato (58°C to 68°C), tapioca (59°C to 69°C) and sweet potato (58°C to 72°C). (J.J.M. Swinkels pg 32 - 38 in STARCH CONVERSION TECHNOLOGY, Eds Van Beynum et al., (1985) Marcel Dekker Inc. New York and The Alcohol Textbook 3rd ED. A Reference for the Beverage, Fuel and Industrial Alcohol Industries, Eds Jacques et al., (1999) Nottingham University Press, UK).

The term "below the gelatinization temperature" refers to a temperature that is less than the gelatinization temperature.

As used herein the term "dry solids content (DS)" refers to the total solids of a milled grain in % on a dry weight basis including moisture.

The term "slurry" refers to an aqueous mixture comprising insoluble solids, (e.g. granular starch).

The term "mash" refers to a mixture of a fermentable substrate in liquid used in the production of a fermented product and is used to refer to any stage of the fermentation from the initial mixing of the fermentable substrate with one or more starch hydrolyzing enzymes and fermenting organisms through the completion of the fermentation run.

The term "fermentation" refers to the enzymatic and anaerobic breakdown of organic substances by microorganisms to produce simpler organic compounds. While fermentation occurs under anaerobic conditions it is not intended that the term be solely limited to strict anaerobic conditions, as fermentation also occurs in the presence of oxygen.

The phrase "simultaneous saccharification and fermentation (SSF)" refers to a process in the production of end products in which a fermenting organism, such as an ethanol producing microorganism, and at least one enzyme, such as a saccharifying enzyme are combined in the same process step in the same vessel.

The term "saccharification" refers to enzymatic conversion of a directly unusable polysaccharide to a mono- or oligosaccharide for fermentative conversion to an end product.

The term "end product" refers to any carbon-source derived product which is enzymatically converted from a fermentable substrate. In some preferred embodiments, the end product is an alcohol, such as ethanol.

As used herein the term "fermenting organism" refers to any microorganism or cell, which is suitable for use in fermentation for directly or indirectly producing an end product.

As used herein the term "ethanol producer" or ethanol producing microorganism" refers to a fermenting organism that is capable of producing ethanol from a mono- or oligosaccharide.

The terms "recovered", "isolated", and "separated" as used herein refer to a protein, cell, nucleic acid or amino acid that is removed from at least one component with which it is naturally associated.

The term "derived" encompasses the terms "originated from", "obtained" or "obtainable from", and "isolated from" and in some embodiments as used herein means that a polypeptide encoded by the nucleotide sequence is produced from a cell in which the nucleotide is naturally present or in which the nucleotide has been inserted.

The term "enzymatic conversion" in general refers to the modification of a substrate by enzyme action.

The term "yield" refers to the amount of end product produced using the methods of the present invention. In some embodiments, the term refers to the volume of the end product, and in other embodiments, the term refers to the concentration of the end product.

As used herein the term "enzyme unit" refers to the amount of enzyme that produces 1 micromole of product per minute under the specified conditions of the assay. For example, in one embodiment, the term "glucoamylase activity unit" (GAU) is defined as the amount of enzyme required to produce 1 g of glucose per hour from soluble starch substrate (4% DS) under assay conditions of 60°C and pH 4.2. In another embodiment, one unit of enzyme activity for a "soluble starch unit (SSU)" is equivalent to the reducing power of 1 mg of glucose released per minute at the specific incubation conditions and is based on the degree of hydrolysis of soluble potato starch substrate (4% DS) by an aliquot of the enzyme sample at pH 4.5, 50°C. DS refers to "dry solids."

As used herein the term "comprising" and its cognates are used in their inclusive sense; that is, equivalent to the term "including" and its corresponding cognates.

"A", "an" and "the" include plural references unless the context clearly dictates otherwise.

Numeric ranges are inclusive of the numbers defining the range.

The headings provided herein are not limitations of the various aspects or embodiments of the invention, which can be had by reference to the specification as a whole.

### Embodiments of the Invention

In plants cells as well as microbial cells, alpha-amylases are also involved in the hydrolysis of starch, and the onset of starch degradation during cereal seed germination is accompanied by large de novo synthesis of alpha-amylases. Thus, the cereal seed has its own endogenous starch hydrolyzing alpha-amylase enzymes that are involved in generating simple sugars to be used in germination and growth.

Under temperature conditions below the temperature of initial starch gelatinization temperature (e.g. less than 80°C), enzyme combinations of starch hydrolyzing enzymes, including those derived from microbial sources and those derived from native plant alpha-amylases, offer an improved approach for hydrolyzing granular starch and obtaining fermentable sugars and other end products.

### Milled plant material -

Plant material comprising granular starch may be obtained from but not limited to wheat, corn, rye, sorghum (milo), rice, millet, barley, triticale, cassava (tapioca), potato, sweet potato, sugar beets, sugarcane, and legumes such as soybean and peas. Preferred plant material includes corn, barley, wheat, rice, milo and combinations thereof. Plant material may include hybrid varieties and genetically modified varieties (e.g. transgenic corn, barley or soybeans comprising heterologous genes). Any part of the plant may be used to as plant material including but not limited to plant parts such as leaves, stems, hulls, husks, tubers, cobs, grains and the like. In one embodiment, whole grain may be used as a source of granular starch. Preferred whole grains include corn, wheat, rye, barley, sorghum and combinations thereof.

Preferably the whole grain is reduced in size by means known in the art including milling (e.g. hammer milling or roller milling); emulsion technology; rotary pulsation; fractionation and the like. In some embodiments, the plant material is ground so that at least 70% will pass through a sieve having a 0.5 mm screen. In some embodiments, at least 90% of the ground plant material will pass through a sieve having a 0.5 mm screen.

In other embodiments, the plant material is fractionated cereal grain, which includes fiber, endosperm and/or germ components. In some embodiments certain fractions will be used in the starch hydrolysis process of the invention. Methods for fractionating plant material such as corn, barley and wheat are known in the art.

### Plant alpha-amylases -

In the invention, an enzyme composition for use in the hydrolysis of starch from a milled plant material is an exogenous plant alpha-amylase and a Trichoderma or Humicola glucoamylase.

Exogenous plant alpha-amylases may be obtained from a variety of plants and plant parts, such as corn, barley, wheat, rice and milo. At least one plant alpha-amylase has been purified and may be obtained commercially from such sources as Sigma-Aldrich. Preferred plant amylases include those derived from barley and milo.

Numerous plant alpha-amylases have been purified and some of these enzymes have been sequenced. Examples of plant alpha-amylases include those found in Arabidopsis (GenBank NP 564977); Rice (Karrer et al., (1992) The Plant J. 2:517-532; and Karrer et al., (1991) Plant Mol. Biol. 16:797-805); maize (US 2005/0138688; Warner et al., (1991) Plant Sci. 78:143-150 and Subbarao et al., (1998) Phytochem. 49:657 - 666); barley (Xavier et al., (2003) Structure 11:973-984; MacGregor et al. (2001) Biochim. Biophys. Acta 1546:1-20; and GenBank X05166).

At temperatures conducted in the present process, it is believed that the endogenous plant alpha-amylases are not inactivated and may also contribute to the hydrolysis of granular starch.

The exogenous plant alpha-amylases according to the invention may be added alone or in combination with other enzymes.

### Glucoamylases -

In the invention, the process includes contacting the milled plant material with a combination of an exogenous plant alpha-amylase and a Trichoderma or Humicola glucoamylase.

Glucoamylases (E.C. 3.2.1.3.) may be derived from the heterologous or endogenous protein expression of bacteria, plants and fungi sources. Preferred glucoamylases are produced by several strains of filamentous fungi and yeast. In particular, glucoamylases secreted from strains of *Aspergillus* and *Trichoderma* are commercially important. Suitable glucoamylases include naturally occurring wild-type glucoamylases as well as variant and genetically engineered mutant glucoamylases. The following glucoamylases are nonlimiting examples of glucoamylases *. Aspergillus niger* G1 and G2 glucoamylase (Boel et al., (1984) EMBO J. 3:1097 - 1102; WO 92/00381, WO 00/04136 and USP 6,352,851); *Aspergillus awamori* glucoamylases (WO 84/02921); *Aspergillus oryzae* glucoamylases (Hata et al., (1991) Agric. Biol. Chem. 55:941 - 949) and *Aspergillus shirousami.* (See Chen et al., (1996) Prot. Eng. 9:499 - 505; Chen et al. (1995) Prot. Eng. 8:575-582; and Chen et al., (1994) Biochem J. 302:275-281).

Glucoamylases are also obtained from strains of *Talaromyces* such as those derived from *T. emersonii, T. leycettanus, T. duponti* and *T. thermophilus* (WO 99/28488; USP No. RE: 32,153; USP No. 4,587,215); strains of *Trichoderma,* such as *T. reesei* and particularly glucoamylases having at least 80%, 85%, 90% and 95% sequence identity to SEQ ID NO: 4 disclosed in US Pat. Pub. No. 2006-0094080; strains of *Rhizopus,* such as *R. niveus and R. oryzae;* strains of *Mucor* and strains of *Humicola,* such as *H. grisea* (See, Boel et al., (1984) EMBO J. 3:1097-1102; WO 92/00381; WO 00/04136; Chen et al., (1996) Prot. Eng. 9:499-505; Taylor et al., (1978) Carbohydrate Res. 61:301-308; USP. 4,514,496; USP 4,092,434; USP 4,618,579; Jensen et al., (1988) Can. J. Microbiol. 34:218 - 223 and SEQ ID NO: 3 of WO 2005/052148). The glucoamylase will have at least 85%, 90%, 92%, 94%, 95%, 96%, 97%, 98% and 99% sequence identity to the amino acid sequence of SEQ ID NO: 3 of WO 05/052148.

Other glucoamylases include those obtained from *Athelia rolfsii* and variants thereof (WO 04/111218).

Enzymes having glucoamylase activity used commercially are produced for example, from *Aspergillus niger* (trade name DISTILLASE, OPTIDEX L-400 and G ZYME G990 4X from Genencor International Inc.) or *Rhizopus* species (trade name CU.CONC from Shin Nihon Chemicals, Japan). Also the commercial digestive enzyme, trade name GLUCZYME from Amano Pharmaceuticals, Japan (Takahashi et al., (1985) J. Biochem. 98:663-671). Additional enzymes include three forms of glucoamylase (E.C.3.2.1.3) of a Rhizopus sp., namely "Gluc1" (MW 74,000), "Gluc2" (MW 58,600) and "Gluc3" (MW 61,400). Also the enzyme preparation GC480 (Genencor International Inc.) finds use.

### Microbially derived alpha-amylase -

In another preferred embodiment of the invention, the process includes contacting milled plant material with a combination of an exogenous plant alpha-amylase, a Trichoderma or Humicola glucoamylase and a microbially derived alpha-amylase.

Any suitable alpha-amylase may be used as a microbial alpha-amylase in the invention. In some embodiments, the alpha-amylase is derived from a bacterial strain and in other embodiments the alpha-amylase is derived from a fungal strain. In further embodiments, the preferred alpha-amylase is a bacterial alpha-amylase. In other embodiments, the alpha-amylase is an acid stable alpha-amylase. Suitable alpha-amylases may be naturally occurring as well as recombinant (hybrid and variants) and mutant alpha-amylases (WO 99/19467 and WO 97/41213). In some preferred embodiments, the alpha-amylase is derived from a *Bacillus* species. Preferred *Bacillus* species include *B. subtilis, B. stearothermophilus, B. lentus, B. licheniformis, B. coagulans,* and *B. amyloliquefaciens* (USP 5,093,257; USP 5,763,385; USP 5,824,532; USP 5,958,739; USP 6,008,026, USP 6,361,809; USP 6,867,031; WO 96/23874; WO 96/39528 and WO 05/001064). Particularly preferred alpha-amylases are derived from *Bacillus* strains *B. stearothermophilus, B. amyloliquefaciens* and *B. licheniformis* ((USP 6,187,576; USP 6,093,562; USP 5,958,739; US 2006/0014265 and WO 99/19467). Such alpha-amylases include wild type, hybrid and variant alpha-amylase enzymes. See Suzuki et al., (1989) J. Biol. Chem. 264:18933-18938 and US 2006/0014265, particularly SEQ ID NOs: 3, 4 and 16. Reference is also made to strains having American Type Culture Collection (ATCC) numbers - ATCC 39709; ATCC 11945; ATCC 6598; ATCC 6634; ATCC 8480; ATCC 9945A and NCIB 8059.

In addition to the bacterial alpha-amylases, fungal alpha-amylases are contemplated Suitable fungal alpha-amylases are derived from filamentous fungal strains such as *Aspergillus,* such as *A. oryzae* and *A. niger* (e.g. FUNGAMYL and CLARASE L), and *Trichoderma, Rhizopus, Mucor,* and *Penicillium.*

Commercially available alpha-amylases contemplated include; SPEZYME AA; SPEZYME FRED; SPEZYME ETHYL; GZYME G997; CLARASE L (Genencor International Inc.); TERMAMYL 120-L, LC, SC and SUPRA (Novozymes Biotech); LIQUOZYME X and SAN SUPER (Novozymes A/S) and ULTRA THIN (/Valley Research).

### Fermenting organisms -

Examples of fermenting organisms are ethanologenic microorganisms or ethanol producing microorganisms such as ethanologenic bacteria which express alcohol dehydrogenase and pyruvate dehydrogenase and which can be obtained from Zymomonas moblis (See e.g. USP 5,000,000; USP 5,028,539, USP 5,424,202; USP 5,514,583 and USP 5,554,520). In additional embodiments, the ethanologenic microorganisms express xylose reductase and xylitol dehydrogenase, enzymes that convert xylose to xylulose. In further embodiments, xylose isomerase is used to convert xylose to xylulose. In particularly preferred embodiments, a microorganism capable of fermenting both pentoses and hexoses to ethanol are utilized. For example, in some embodiments the microorganism may be a natural or non-genetically engineered microorganism or in other embodiments the microorganism may be a recombinant microorganism.

In some embodiments, the preferred fermenting microorganisms include bacterial strains from *Bacillus, Lactobacillus, E. coli, Erwinia, Pantoea* (e.g., P. citrea), *Pseudomonas* and *Klebsiella* (e.g. K. oxytoca). (See e.g. USP 5,028,539, USP 5,424,202 and WO 95/13362). The fermenting microorganism used in the fermenting step will depend on the end product to be produced.

In further preferred embodiments, the ethanol-producing microorganism is a fungal microorganism, such as a yeast and specifically Saccharomyces such as strains of *S*. *cerevisiae* (USP 4,316,956). A variety of *S. cerevisiae* are commercially available and these include but are not limited to FALI (Fleischmann's Yeast), SUPERSTART (Alltech), FERMIOL (DSM Specialties), RED STAR (Lesaffre) and Angel alcohol yeast (Angel Yeast Company, China).

### Secondary Enzymes -

While the invention include exogenous plant alpha-amylases, Trichoderma or Humicola glucoamylases and optionally microbially derived alpha-amylases, further enzymes may be included in the contacting step and/or the fermenting step along with the fermenting microorganism and other components. The additional enzymes include without limitation, cellulases, hemicellulases, xylanase, proteases, phytases, pullulanases, lipases, cutinases, pectinases, beta-glucanases, cyclodextrin transglycosyltransferases, beta-amylases and combinations thereof.

Other glucoamylases, which may be included with the alpha amylase and Trichoderma or Humicola glucoamylase, may be derived from the heterologous or endogenous protein expression of bacteria, plants and fungi. Preferred glucoamylases are produced by several strains of filamentous fungi and yeast. In particular, glucoamylases obtained from strains of *Aspergillus, (A. niger,* See, Boel et al., (1984) EMBO J. 3:1097 - 1102; WO 92/00381 and USP 6,352,851); *A. oryzae,* See, Hata et al., (1991) Agric. Biol. Chem. 55:941-949 and *A. shirousami,* See, Chen et al., (1996) Prot. Eng. 9:499 - 505). *Trichoderma, Rhizopus (R. niveus* and *R. oryzea), Humicola (H. grisea* See, Boel et al., (1984) EMBO J. 3: 1097-1102; USP 4,514,496 and USP 4,092,434), *Talaromyces* (*T. emersonii, T. thermophilus* and *T. duponti,* See, WO 99/28488 and USP 4,587,215) and *Athelia (A. rolfsii,* See, WO 04/111218) may be useful. Enzymes having glucoamylase activity used commercially are produced for example from *Aspergillus niger* (trade name DISTILLASE, OPTIDEX L-400 and G ZYME G990 4X from Genencor International Inc.).

A composition comprising a glucoamylase and an alpha amylase, which is useful according to the invention is STARGEN™ 001, which is a blend of an *Aspergillus kawachi* alpha amylase having GSH activity and an *Aspergillus niger* glucoamylase (available commercially from Genencor International, Inc).

In some embodiments the additional enzyme is an alpha amylase such as a bacterial or fungal alpha amylase, and in other embodiments the alpha amylase is a derivative, mutant or variant of a fungal or bacterial alpha amylase. Non-limiting examples of alpha amylases useful in combination with a starch hydrolyzing enzyme having GSH activity according to the invention are those derived from *Bacillus, Aspergillus, Trichoderma, Rhizopus, Fusarium, Penicillium, Neurospora* and *Humicola.*

Some preferred additional alpha amylases are derived from *Bacillus* including *B. licheniformis, B. lentus, B. coagulans, B. amyloliquefaciens. B. stearothermophilus, B subtilis,* and hybrids, mutants and variants thereof (USP 5,763,385; USP 5,824,532; USP 5,958,739; USP 6,008,026 and USP 6,361,809). Some of these amylases are commercially available e.g., TERMAMYL and SUPRA available from Novo Nordisk A/S, ULTRATHIN from Diversa, LIQUEZYME SC from Novo Nordisk A/S and SPEZYME FRED, SPEZYME ETHYL and GZYME G997 available from Genencor International, Inc.

In another embodiment, the invention will include the addition of a phytase. A phytase is an enzyme that is capable of liberating at least one inorganic phosphate from an inositol hexaphosphate. Phytases are grouped according to their preference for a specific position of the phosphate ester group on the phytate molecule at which hydrolysis is initiated, (e.g., as 3-phytases (EC 3.1.3.8) or as 6-phytases (EC 3.1.3.26)). A typical example of phytase is myo-inositol-hexakiphosphate-3-phosphohydrolase. Phytases may be obtained from microorganisms such as fungal and bacterial organisms. Some of these microorganisms include *e.g. Aspergillus. (e.g., A. niger, A. terreus,* and *A. fumigalus), Myceliophthora (M. thermophila), Talaromyces (T. thermophilus) Trichoderma spp (T. reesei).* and *Thermomyces* (WO 99/49740). Also phytases are available from *Penicillium* species, e.g., *P. hordei* (ATCC No. 22053), *P. piceum* (ATCC No. 10519), or *P. brevi-compactum* (ATCC No. 48944). See, for example USP 6,475,762. In addition, phytases are available from *Peniophora, E. coli, Citrobacter, Enterbacter* and *Buttiauxella.* Preferably, the phytase from *Buttiauxella* is the BP-17 phytase (see United States patent application US 2008220498, filed March 6, 2007, entitled VARIANT *BUTTIAUXELLA SP.* PHYTASES HAVING ALTERED PROPERTIES. Commercial phytases are available such as NATUPHOS (BASF), RONOZYME P (Novozymes A/S), PHZYME (Danisco A/S, Diversa) and FINASE (AB Enzymes). The method for determining microbial phytase activity and the definition of a phytase unit has been published by Engelen et al. (1994) J. of AOAC International, 77: 760 - 764.

Cellulases may also be incorporated with the alpha amylase and glucoamylase. Cellulases are enzyme compositions that hydrolyze cellulose (β-1, 4-D-glucan linkages) and/or derivatives thereof, such as phosphoric acid swollen cellulose. Cellulases include the classification of exo-cellobiohydrolases (CBH), endoglucanases (EG) and β-glucosidases (BG) (EC3.2.191, EC3.2.1.4 and EC3.2.1.21). Examples of cellulases include cellulases from *Penicillium, Trichoderma, Humicola, Fusarium, Thermomonospora, Cellulomonas, Clostridium* and *Aspergillus.* Commercially available cellulases sold for feed applications are beta-glucanases such as ROVABIO (Adisseo), NATUGRAIN (BASF), MULTIFECT BGL (Danisco Genencor) and ECONASE (AB Enzymes).

Xylanases may also be included. Xylanases (e.g. endo-β-xylanases (E.C. 3.2.1.8), which hydrolyze the xylan backbone chain may be from bacterial sources, such as *Bacillus, Streptomyces, Clostridium, Acidothermus, Microtetrapsora or Thermonospora.* In addition xylanases may be from fungal sources, such as *Aspergillus, Trichoderma, Neurospora, Humicola, Penicillium* or *Fusarium.* (See, for example, EP473 545; USP 5,612,055; WO 92/06209; and WO 97/20920). Commercial preparations include MULTIFECT and FEEDTREAT Y5 (Danisco Genencor), RONOZYME WX (Novozymes A/S) and NATUGRAIN WHEAT (BASF).

Proteases may also be included. Proteases may be derived from *Bacillus* such as B. *amyloliquefaciens, B. lentus, B. licheniformis,* and *B. subtilis.* These sources include subtilisin such as a subtilisin obtainable from *B. amyloliquefaciens* and mutants thereof (USP 4,760,025). Suitable commercial protease includes MULTIFECT P 3000 (Danisco Genencor) and SUMIZYME FP (Shin Nihon). Proteases are also derived from fungal sources such as *Trichoderma* (for example NSP-24), *Aspergillus, Humicola* and *Penicillium.*

Additional enzymes, which may be included , are α-galactosidases, pectinases, mannanases, lipases, cyclodextrin glycosyl transferases (CGTases), hemicellulases, oxidases, oxido-reductases, pullulanases, beta amylases, (E.C. 3.2.1.2) and esterases.

In some preferred embodiments, combinations of two or more enzymes selected from alpha amylases, glucoamylases, phytases, cellulases, hemicellulases, and xylanases will be included.

### Process Steps -

In some embodiments the milled plant material comprising granular starch is mixed with an aqueous solution to obtain a slurry. The slurry may have a DS of between 5 - 60%; 10 - 50%; 15 - 45%; 15- 30%; 20 - 45%; 20 - 30% and also 25 - 40%. The slurry is contacted with an exogenous plant alpha-amylase, a glucoamylase and optionally a microbial alpha-amylase under suitable conditions to produce fermentable sugars.

The pH range of the contacting step is between pH 3.0 to 7.0; also between pH 3.5 to 6.5; also between pH 4.0 to 6.0 and further between pH 4.0 to 5.5. The slurry is held in contact with the enzymes at a temperature below the starch gelatinization temperature of the granular starch in the milled plant material. In some embodiments, the temperature is held between 25°C and 75°C; in other embodiments, the temperature is held between 30°C and 70°C; between 30°C and 65°C; between 40°C and 65°C; between 55°C and 70°C, between 60°C and 65°C; between 55°C and 65°C and between 55°C and 68°C. In further embodiments, the temperature is at least 25°C 30°C, 35°C, 40°C, 45°C, 48°C, 50°C, 53°C, 55°C, 58°C, 60°C, 63°C, 65°C and 68°C. In other embodiments, the temperature is not greater than 65°C, 68°C, 70°C, 73°C, 75°C and 80°C.

The initial starch gelatinization temperature ranges for a number of granular starches which may be used in accordance with the processes herein include barley (52°C to 59°C), wheat (58°C to 64°C), rye (57°C to 70°C), corn (62°C to 72°C), high amylose corn (67°C to 80°C), rice (68°C to 77°C), sorghum (68°C to 77°C), potato (58°C to 68°C), tapioca (59°C to 69°C) and sweet potato (58°C to 72°C). (J.J.M. Swinkels pg 32 - 38 in STARCH CONVERSION TECHNOLOGY, Eds Van Beynum et al., (1985) Marcel Dekker Inc. New York and The Alcohol Textbook 3rd ED. A Reference for the Beverage, Fuel and Industrial Alcohol Industries, Eds Jacques et al., (1999) Nottingham University Press, UK).

In the contacting step, the slurry may be held in contact with the enzymes for a period of 2 hrs to 240 hrs; also for 2 hrs to 120 hrs; also for 5 hrs to 90 hrs; for 5 hrs to 72 hrs; and 5 hrs to 48 hrs.

The effective concentration of the alpha-amylase used in the contacting step will vary according to the specific process conditions and granular starch used. However, in general the amount of alpha-amylase used will be in the range of 0.001 to 50 AAU/g DS, 0.01 to 30 AAU/g DS, 0.01 to 10 AAU/g DS and also 0.05 to 5.0 AAU/g DS.

In some embodiments, the effective dose of an alpha-amylase in the contacting step and/or fermentation step will be 0.01 to 25 SSU/g DS; also 0.01 to 15 SSU/g DS; also 0.05 to 10 SSU/g DS; also 0.1 to 10 SSU/g DS; also 0.1 to 10 SSU/g DS and 0.5 to 5 SSU/g DS.

In some embodiments, the effective dose of a glucoamylase for the contacting step and/or the fermentation step will be in the range of 0.01 to 20 GAU/g DS; also 0.01 to 15 GAU/g DS; also 0.05 to 10 GAU/g DS; also 0.1 to 10 GAU/g DS and even 0.5 to 5 GAU/g DS.

During the contacting step between 20 - 95% of the granular starch is solubilized to produce fermentable sugars such as oligosaccharides. In some embodiments greater than 40%, greater than 50%, greater than 60%, greater than 70%, greater than 80%, and greater than 90% of the starch is solubilized. In some embodiments the solubilized starch comprises greater than 10%, 15%, 20%, 25%, 30%, 35%, 40%, 45%, 50%, 55%, 60%, 65%, 70%, 75% and 80% glucose.

In some embodiments, the mash comprising fermentable sugars may be further converted to end products such as high fructose sugars. In other embodiments the fermentable sugars are subjected to fermentation with fermenting microorganisms. The contacting step and the fermenting step may be preformed simultaneously in the same reaction vessel or sequentially. In general, fermentation processes are described in The Alcohol Textbook 3rd ED, A Reference for the Beverage, Fuel and Industrial Alcohol Industries, Eds Jacques et al., (1999) Nottingham University Press, UK.

In some preferred embodiments, the mash is fermented with a yeast at temperatures in the range of 15 to 40°C, 20 to 38°C, and also 25 to 35°C; at a pH range of pH 3.0 to 6.5; also pH 3.0 to 6.0; pH 3.0 to 5.5, pH 3.5 to 5.0 and also pH 3.5 to 4.5 for a period of time of 5 hrs to 120 hours, preferably 12 to 120 and more preferably from 24 to 90 hours to produce an alcohol product, preferably ethanol.

Yeast cells are generally supplied in amounts of 10⁴ to 10¹², and preferably from 10⁷ to 10¹⁰ viable yeast count per ml of fermentation broth. The fermentation will include in addition to a fermenting microorganisms (e.g. yeast) nutrients, optionally acid and additional enzymes. In some embodiments, in addition to the raw materials described above, fermentation media will contain supplements including but not limited to vitamins (e.g. biotin, folic acid, nicotinic acid, riboflavin), cofactors, and macro and micro-nutrients and salts (e.g. (NH₄)₂SO₄; K₂HPO₄; NaCl; MgSO₄; H₃BO₃; ZnCl₂; and CaCl₂).

In some preferred embodiments, the milled plant material includes barley, milo, corn and combinations thereof, and the contacting and fermenting steps are conducted simultaneously at a pH range of 3.5 to 5.5, a temperature range of 30 - 45°C, and for a period of time of 48 to 90 hrs, wherein at least 50% of the starch is solubilized.

### Recovery of alcohol and other end products -

The preferred end product of the instant fermentation process is an alcohol product, preferably ethanol. The end product produced according to the process may be separated and/or purified from the fermentation media. Methods for separation and purification are known, for example by subjecting the media to extraction, distillation and column chromatography. In some embodiments, the end product is identified directly by submitting the media to high-pressure liquid chromatography (HPLC) analysis.

In further embodiments, the mash may be separated by for example centrifugation into the liquid phase and solids phase and end products such as alcohol and solids recovered. The alcohol may be recovered by means such as distillation and molecular sieve dehydration or ultra filtration.

In some embodiments, the yield of ethanol will be greater than 8%, 10%, 12%, 14%, 16% and 18% by volume. The ethanol obtained according to process of the invention may be used as a fuel ethanol, potable ethanol or industrial ethanol.

The end product may include the fermentation co-products such as distillers dried grains (DDG) and distiller's dried grain plus solubles (DDGS), which may be used as an animal feed.

By use of appropriate fermenting microorganisms as known in the art, the fermentation end product may include without limitation glycerol, 1,3-propanediol, gluconate, 2-keto-D-gluconate, 2,5-diketo-D-gluconate, 2-keto-L-gulonic acid, succinic acid, lactic acid, amino acids and derivatives thereof. More specifically when lactic acid is the desired end product, a *Lactobacillus* sp. (*L. casei*) may be used; when glycerol or 1,3-propanediol are the desired end-products *E.coli* may be used; and when 2-keto-D-gluconate, 2,5-diketo-D-gluconate, and 2-keto-L-gulonic acid are the desired end products, *Pantoea citrea* may be used as the fermenting microorganism. The above enumerated list are only examples and one skilled in the art will be aware of a number of fermenting microorganisms that may be appropriately used to obtain a desired end product.

### EXPERIMENTAL

The following examples are provided in order to demonstrate and further illustrate certain preferred embodiments and aspects of the present invention and are not to be construed as limiting the scope thereof. Indeed, it is contemplated that these teachings will find use in further optimizing the process systems described herein.

In the disclosure and experimental section which follows, the following abbreviations apply: GA (glucoamylase); AnGA (glucoamylase obtained from *Aspergillus niger);* HGA (glucoamylase derived from *Humicola grisea,* see SEQ ID NO: 3 of WO 2005/052148); STARGEN 001 (glucoamylase and alpha-amylase blend, Genencor International, Inc.); TrGA (glucoamylase derived from *Trichoderma,* see SEQ ID NO: 4 of US 2006/0094080); Barley AA (Barley alpha-amylase at 1.7 AAU/mg purchased from Sigma (Sigma A2771-1MU); wt% (weight percent); °C (degrees Centigrade); H₂O (water); dH₂O (deionized water); dIH₂O (deionized water, Milli-Q filtration); g or gm (grams); µg (micrograms); mg (milligrams); kg (kilograms); µL (microliters); ml and mL (milliliters); mm (millimeters); µm (micrometer); M (molar); mM (millimolar); µM (micromolar); U (units); MW (molecular weight); sec (seconds); min(s) (minute/minutes); hr(s) (hour/hours); W/V (weight to volume); W/W (weight to weight); VN (volume to volume); Genencor (Genencor International, Inc., Palo Alto, CA); MT (Metric ton); and ETOH (ethanol).

### The following assays were used in the examples below:

The activity of alpha-amylase is expressed as alpha-amylase units (AAU) and enzyme activity was determined by the rate of starch hydrolysis, as reflected in the rate of decrease of iodine-staining capacity, which was measured spectrophotometrically. One AAU of bacterial alpha-amylase activity is the amount of enzyme required to hydrolyze 10 mg of starch per min under standardized conditions.

Alpha-amylase activity made also determined as soluble starch unit (SSU) and is based on the degree of hydrolysis of soluble potato starch substrate (4% DS) by an aliquot of the enzyme sample at pH 4.5, 50°C. The reducing sugar content is measured using the DNS method as described in Miller, G. L. (1959) Anal. Chem. 31:426 - 428. One unit of the enzyme activity (SSU) is equivalent to the reducing power of 1 mg of glucose released per minute at the specific incubation conditions.

Glucoamylase activity was measured using a well-known assay which is based on the ability of glucoamylase to catalyze the hydrolysis of p-nitrophenyl-alpha-D-glucopyranoside (PNPG) to glucose and p-nitrophenol. At an alkaline pH, the nitrophenol; forms a yellow color that is proportional to glucoamylase activity and is monitored at 400nm and compared against an enzyme standard measured as a GAU.

One "Glucoamylase Activity Unit" (GAU) is the amount of enzyme that will produce 1 gm of reducing sugar, calculated as glucose per hour from a soluble starch substrate (4% DS) at pH 4.2 and 60°C.

Brix, the measurement of total solubilized solid content at a given temperature was determined by measurement with a Refractometer.

Determination of total starch content: The enzyme-enzyme starch liquefaction and saccharification process was used to determine the total starch content. In a typical analysis, 2 g of dry sample was taken in a 100 ml Kohlraucsh flask and 45 ml of MOPS buffer, pH 7.0 was added. The slurry was well stirred for 30 min. SPEZYME FRED (1:50 diluted in water) (Genencor), 1.0 ml was added and heated to boiling for 3 - 5 min. The flask was placed in an autoclave maintained at 121 °C for 15 min. After autoclaving the flask was placed in a water bath at 95°C and 1 ml of 1:50 diluted SPEZYME FRED was added and incubated for 45 min. The pH was adjusted to pH 4.2 and the temperature was reduced to 60°C. This was followed by addition of 20 ml acetate buffer, pH 4.2. Saccharification was carried out by adding 1.0 ml of 1:100 diluted OPTIDEX L-400 (Genencor) and the incubation was continued for 18 hr at 60°C. The enzyme reaction was terminated by heating at 95°C for 10 min. The total sugar composition was determined by HPLC analysis using glucose as a standard. The soluble starch hydrolysate from water extraction of a sample at room temperature without enzymatic treatment was subtracted from the total sugar. Starch and moisture content of barley flour was further confirmed by external analysis (Rock River Labs, Watertown, WI). From this, the % starch conversion to ethanol can be calculated knowing the total starch content, the total starch solubilized to ethanol (starch solubilized x 100/total starch).

Ethanol and carbohydrate determinations of the samples were determined using the HPLC method as follows: a 1.5 mL Eppendorf centrifuge tube was filled with fermentor mash and cooled on ice for 10 min; the sample tube was centrifuged for 1 min in an Eppendorf table top centrifuge; a 0.5 mL sample of the supernatant was transferred to a test tube containing 0.05 mL of 1.1N H₂SO₄ and allowed to stand for 5 min; 5.0 mL of water was added to the test tube and then the sample was filtered into a HPLC vial through 0.2 µm Nylon Syringe Filter; and run on HPLC. The HPLC conditions included:

Ethanol System: Column: Phenomenex Rezex Organic Acid Column (RHM-Monosaccharide) #00H 0132-KO (Equivalent to Bio-Rad 87H); Column Temperature: 60°C; Mobile Phase: 0.01 N H₂SO₄; Flow Rate: 0.6 mL/min; Detector: RI; and Injection Volume: 20 µL.

Carbohydrate System: Column: Phenomenex Rezex Carbohydrate (RCM-Monosaccharide) #00H-0130-KO (Equivalent to Bio-Rad 87H); Column Temperature: 70°C ; Mobile Phase: Nanopure DI H₂O; Flow Rate: 0.8 mL/min; Detector: RI; Injection Volume: 10 µL (3% DS material). The column separated based on the molecular weight of the saccharides, which are designated as DP1 (glucose); DP2 (disaccharides); DP3 (trisaccharides) and DP > 3 (oligosaccharide sugars having a degree of polymerization greater than 3).

### EXAMPLE 1

### Starch Hydrolysis and Ethanol Production from Granular Starch of Ground Barley Grain

Barley grains were purchased from Whole Foods and ground (0.5mm sieve) into flour. Moisture content of the barley flour was 11.1%. Starch content of the barley flour was 57.9%. 32 g of ground barley and 68 g water were added to a 250 screw top bottle. The slurry was adjusted to pH 4.2 and urea (Bio-Rad) was added at 400 ppm. The slurry was exposed to various enzyme treatments at 1 U/g DS of substrate. 200 ul of 10% yeast was added as the final component. The slurry was incubated in a water bath at 32°C and moderately stirred (100 ppm) by a stir plate. A sample was taken approximately every 12 hrs for the duration of 72 total hrs. The sample (about 8 ml) was centrifuged at 3500 rpm for 30 min and 2 ml of supernatant was collected and stored at -80°C for HPLC processing.

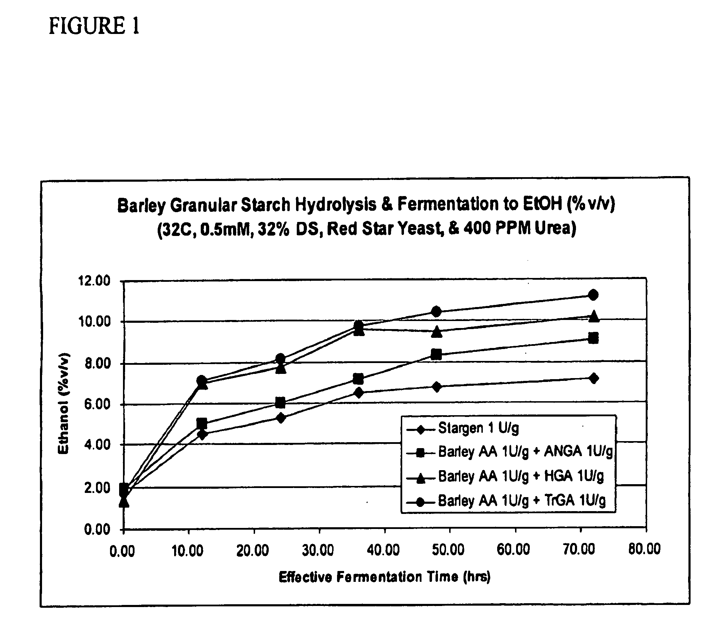

Figure 1 illustrates the hydrolysis of barley granular starch, measured by ethanol production over time, improved with the addition of exogenous plant alpha-amylase as compared to the use of fungal alpha-amylase (STARGEN 001).

**Table 1**

| Sample | Barley AA 32U (mg) | GA 32U (uL) | Combined enzymes (mg) | % Starch conversion to Ethanol | Total % starch solubilized |
|---|---|---|---|---|---|
| STARGEN 001 | N/A | N/A | 65.3 | 50.8 | 65 |
| Barley AA + AnGA | 19 | 61.3 | N/A | 64.8 | 82 |
| Barley AA + TrGA | 19 | 57.3 | N/A | 79.4 | 100 |
| Barley AA + HGA | 19 | 84 | N/A | 72.5 | 94 |
| No Barley AA + HGA | N/A | 84 | N/A | 61.3 | 78 |
| Killed Barley AA + HGA | 19 (50 uL of 1N H₂SO₄ & boil for 5 min | 84 | N/A | 58.4 | 74 |

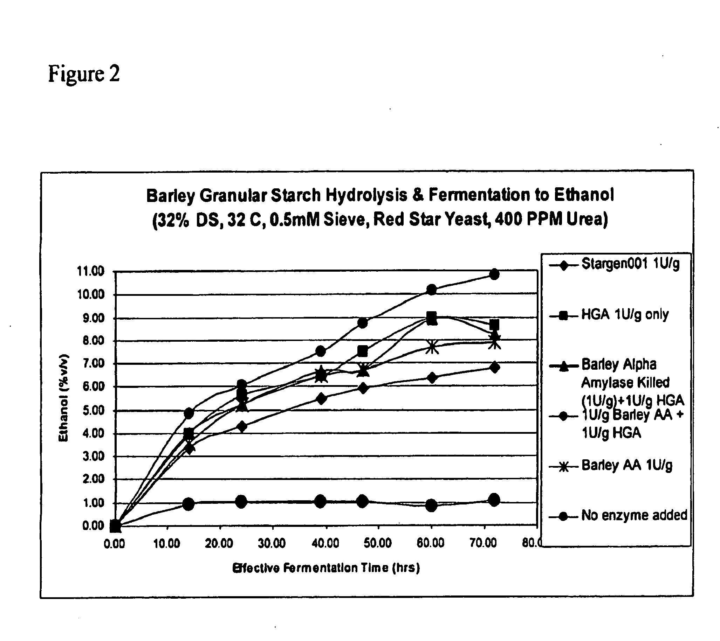

Figure 2, illustrates that endogenous plant alpha-amylase found in the grain flour is active, but it is not active in sufficient concentration to effectively hydrolyze its own starch for further fermentation. There is almost no difference in ethanol production between the acid/heat killed alpha-amylase and control which is without addition of exogenous barley alpha-amylase. These results confirm the role of exogenously added barley alpha-amylase. Addition of exogenous barley alpha-amylase increased the production of ethanol by 20% (v/v) as compared to non-supplemented reactions that relied on endogenous alpha-amylase present in the ground barley seed flour. Addition of exogenous barley alpha-amylase without glucoamylase results in about 8% (v/v) ethanol production. When neither exogenous barley alpha-amylase nor other starch hydrolyzing enzymes were added only 1% (v/v) ethanol was produced during the fermentation. Note that this figure also shows the difference in activity between the two glucoamylases.

## Claims

1. A method of hydrolyzing starch from milled plant material comprising granular starch, said method comprising:
a) contacting milled plant material with an exogenous plant alpha-amylase, and a *Trichoderma* or *Humicola* glucoamylase at a temperature below the starch gelatinization temperature of the granular starch to produce oligosaccharides, and
b) producing fermentable sugars.

2. The method according to claim 1 further comprising:
c) fermenting the fermentable sugars in the presence of fermenting microorganisms at a temperature of between 10°C and 40°C for a period of time of 10 hours to 250 hours to produce alcohol.

3. The method according to claim 1 or claim 2, wherein:
(i) the milled plant material is a slurry comprising granular starch and the slurry has a DS of between 5 and 60%; or
(ii) the milled plant material is a slurry comprising granular starch and the slurry has a DS of between 25 and 40%; or
(iii) the milled plant material is a slurry comprising granular starch and the slurry has a DS of between 15 and 45%

4. The method according to any one of claims 1 to 3, wherein the alpha amylase is added at between 0.001 and 30 AAU/DS.

5. The method according to any one of the preceding claims, wherein said contacting step further comprises contacting the milled plant material with a microbial alpha-amylase.

6. The method according to claim 1, wherein the temperature is between 50° and 70°C.

7. The method according to any one of the preceding claims, wherein:
(i) the plant alpha-amylase is an alpha-amylase selected from the group consisting of barley, wheat, rice corn, rye, sorghum, rice, mullet, triticale, cassava, potato, sweet potato, sugar beet, sugarcane, soybean and pea; and/or
(ii) the plant material is corn milo, barley, wheat, rice or combinations thereof.

8. The method according to claim 7, wherein the plant material is fractionated com.

9. The method according to claim 2, wherein the alcohol is ethanol.

10. The method according to claim 9 further comprising recovering the ethanol.

11. A process for producing ethanol comprising,
a) contacting a slurry comprising granular starch obtained from plant material with an exogenous plant alpha-amylase capable of solubilizing granular starch at a temperature below the starch gelatinization temperature of the granular starch for a period of 5 minutes to 24 hours and a *Trichoderma* or *Humicola* glucoamylase;
b) obtaining a substrate; and
c) fermenting the substrate in the presence of a fermenting microorganism at a temperature of between 10°C and 40°C for a period of 10 hours to 250 hours to produce ethanol.

12. The process according to claim 11 further comprising one or more of the following steps:
(i) recovering the ethanol;
(ii) contacting the slurry with a microbial alpha amylase;
(iii) clarifying the substrate before the fermenting step;
(iv) adding additional enzymes to the contacting step; and/or
(v) contacting the substrate with an aqueous solution comprising backset to dilute the %DS prior to the fermentation step.

13. The process according to claim 11 or claim 12, wherein;
(i) the alpha-amylase is an alpha-amylase selected from the group consisting of barley, wheat, rice corn, rye, sorghum, rice, millet, triticale, cassava, potato, sweet potato, sugar beet, sugarcane, soybean and pea; and /or
(ii) the granular starch is obtained from corn, milo, barley, wheat, rice or combinations thereof.

14. The process according to any one of claims 11 to 13, wherein the contacting step is conducted at a temperature of between 50°C and 70°C.

15. The process according to any one of claims 11 to 14, wherein:
(a) the additional enzymes are selected from the group of phytases, proteases, cellulases and/or hemicellulases; or
(b) the additional enzyme is a phytase and/or a protease; or
(c) the slurry has between 5 - 60% DS granular starch; or
(d) the %DS granular starch is between 20-40%DS.

## Patentansprüche

1. Verfahren des Hydrolysierens von Stärke aus gemahlenem Pflanzenmaterial, das Stärkegranulat umfasst, wobei das Verfahren Folgendes umfaßt:
a) Kontaktieren von gemahlenem Pflanzenmaterial mit einer exogenen Pflanzen-a-Amylase und einer Trichoderma- oder Humicola-Glucoamylase bei einer Temperatur unter der Stärke-Gelatinierungstemperatur des Stärkegranulats, um Oligosaccharide herzustellen, und
b) Herstellung von fermentierbaren Zuckern.

2. Verfahren nach Anspruch 1, weiters umfassend:
c) Fermentieren der fermentierbaren Zucker in der Gegenwart von Fermentierungsmikroorganismen bei einer Temperatur zwischen 10 °C und 40 °C für eine Zeitspanne von 10 Stunden bis 250 Stunden, um Alkohol zu erzeugen.

3. Verfahren nach Anspruch 1 oder Anspruch 2, worin:
(i) das gemahlene Pflanzsnmaterial eine Aufschlämmung ist, die Stärkegranulat umfasst, und die Aufschlämmung einen DS zwischen 5 und 60 % aufweist; oder
(ii) das gemahlene Pflanzenmaterial eine Aufschlämmung ist, die Stärkegranulat umfasst, und die Aufschlämmung einen DS zwischen 25 und 40 % aufweist; oder
(iii) das gemahlene Pflanzenmaterial eine Aufschlämmung ist, die Stärkegranulat umfasst, und die Aufschlämmung einen DS zwischen 15 und 45 % aufweist.

4. Verfahren nach einem der Ansprüche 1 bis 3, worin die α-Amylase zu 0,001 bis 30 AAU/DS hinzugefügt wird.

5. Verfahren nach einem der vorangegangenen Ansprüche, worin der Kontaktierungsschritt weiters das Kontaktieren des gemahlenen Pflanzenmaterials mit einer mikrobiellen α-Amylase umfasst.

6. Verfahren nach Anspruch 1, worin die Temperatur zwischen 50 °C und 70 °C beträgt.

7. Verfahren nach einem der vorangegangenen Ansprüche, worin:
(i) die Pflanzen-α-Amylase eine α-Amylase ist, die aus der Gruppe ausgewählt ist, die aus Gerste, Weizen, Reiskorn, Roggen, Sorghum, Reis, Hirse, Triticale, Maniok, Kartoffel, Süßkartoffel, Zuckerrübe, Zuckerrohr, Sojabohne und Erbse besteht; und/oder
(ii) das Pflanzenmaterial Mais, Milo, Gerste, Weizen, Reis oder Kombinationen davon ist.

8. Verfahren nach Anspruch 7, worin das Pflanzenmaterial fraktionierter Mais ist.

9. Verfahren nach Anspruch 2, worin der Alkohol Ethanol ist.

10. Verfahren nach Anspruch 9, das weiters die Gewinnung des Ethanols umfasst.

11. Verfahren zur Herstellung von Ethanol, umfassend
(a) Kontaktieren einer Aufschlämmung, die Stärkegranulat umfasst, das aus Pflanzenmaterial erhalten wurde, mit einer exogenen Pflanzen-α-Amylase, die in der Lage ist, Stärkegranulat zu solubilisieren, bei einer Temperatur, die sich unterhalb der Stärke-Gelatinierungstemperatur des Stärkegranulats befindet, für eine Zeitspanne von 5 Minuten bis 24 Stunden und einer Trichoderma- oder Humicola-Glucoamylase;
(b) Gewinnen eines Substrats; und
(c) Fermentieren des Substrats in der Gegenwart eines Fermentierungsmikroorganismus bei einer Temperatur zwischen 10 °C und 40 °C für eine Zeitspanne von 10 Stunden bis 250 Stunden, um Ethanol zu erzeugen.

12. Verfahren nach Anspruch 11, das weiters einen oder mehrere der folgenden Schritte umfasst:
(i) Gewinnung des Ethanols;
(ii) Kontaktieren der Aufschlämmung mit einer mikrobiellen α-Amylase;
(iii) Klärung des Substrats vor dem Fermentierungsschritt;
(iv) Hinzufügen von zusätzlichen Enzymen zu dem Kontaktierungsschritt; und/oder
(v) Kontaktieren des Substrats mit einer wässrigen Lösung, welche eine Rückströmung umfasst, um den % DS zu verdünnen, vor dem Fermentierungsschritt.

13. Verfahren nach Anspruch 11 oder Anspruch 12, worin:
(i) die α-Amylase eine α-Amylase ist, die aus der Gruppe ausgewählt ist, die aus Gerste, Weizen, Reiskorn, Roggen, Sorghum, Reis, Hirse, Triticale, Maniok, Kartoffel, Süßkartoffel, Zuckerrübe, Zuckerrohr, Sojabohne und Erbse besteht; und/oder
(ii) das Stärkegranulat aus Mais, Milo, Gerste, Weizen, Reis oder Kombinationen davon erhalten wird.

14. Verfahren nach einem der Ansprüche 11 bis 13, worin der Kontaktierungsschritt bei einer Temperatur zwischen 50 °C und 70 °C durchgeführt wird.

15. Verfahren nach einem der Ansprüche 11 bis 14, worin:
(a) die zusätzlichen Enzyme aus der Gruppe von Phytasen, Proteasen, Cellulasen und/oder Hemicellulasen ausgewählt sind; oder
(b) das zusätzliche Enzym eine Phytase und/oder eine Protease ist; oder
(c) die Aufschlämmung zwischen 5-60 % DS Stärkegranulat aufweist; oder
(d) der % DS des Stärkegranulats zwischen 20-40 % DS beträgt.

## Revendications

1. Méthode pour hydrolyser l'amidon d'un matériau de plante broyée comprenant un amidon granulaire, ladite méthode comprenant:
a) mettre en contact le matériau de plante broyé avec une alpha-amylase de plante exogène et une glucoamylase de *Trichoderma* ou *Humicola* à une température en dessous de la température de gélatinisation de l'amidon granulaire pour produire des oligosaccharides, et
b) produire des sucres fermentables.

2. Méthode selon la revendication 1, comprenant en outre:
c) faire fermenter les sucres fermentables en présence de microorganismes de fermentation à une température entre 10°C et 40°C pendant une période de temps de 10 heures à 250 heures pour produire de l'alcool.

3. Méthode selon la revendication 1 ou la revendication 2, dans laquelle
(i) le matériau de plante broyé est une boue comprenant un amidon granulaire, et la boue a un DS entre 5 et 60%; ou
(ii) le matériau de plante broyé est une boue comprenant de l'amidon granulaire, et la boue a un DS entre 25 et 40%; ou
(iii) le matériau de plante broyé est une boue comprenant de l'amidon granulaire, et la boue a un DS entre 15 et 45%.

4. Méthode selon l'une quelconque des revendications 1 à 3, dans laquelle l'alpha amylase est ajoutée entre 0,001 et 30 AAU/DS.

5. Méthode selon l'une quelconque des revendications précédentes, dans laquelle ladite étape de mise en contact comprend en outre la mise en contact du matériau de plante broyé avec une alpha-amylase microbienne.

6. Méthode selon la revendication 1, dans laquelle la température est entre 50° et 70°C.

7. Méthode selon l'une quelconque des revendications précédentes, dans laquelle
(i) l'alpha-amylase de plante est une alpha-amylase sélectionnée dans le groupe consistant en orge, blé, mélange riz-maïs, seigle, sorgho, riz, millet, triticale, manioc, pomme de terre, patates, betterave sucrière, canne à sucre, soja et pois; et/ou
(ii) le matériau de plante sont des grains, sorgho, orgue, blé, riz ou leurs combinaisons.

8. Méthode selon la revendication 7, dans laquelle le matériau de plante sont des grains fractionnés.

9. Méthode selon la revendication 2, dans laquelle l'alcool est l'éthanol.

10. Méthode selon la revendication 9, comprenant en outre la récupération de l'éthanol.

11. Procédé de production d'éthanol comprenant
a) mettre en contact une boue comprenant un amidon granulaire obtenu d'un matériau de plante avec une alpha-amylase de plante exogène apte à solubiliser l'amidon granulaire à une température en dessous de la température de gélatinisation de l'amidon granulaire pendant une période de 5 minutes à 24 heures et une glucoamylase de *Trichoderma* ou *Humicola;*
b) obtenir un substrat; et
c) faire fermenter le substrat en présence d'un microorganisme de fermentation à une température entre 10°C et 40°c pendant une période de 10 heures à 250 heures pour produire de l'éthanol.

12. Méthode selon la revendication 11, comprenant en outre une ou plusieurs des étapes suivantes :
(i) récupérer l'éthanol;
(ii) mettre en contact la boue avec une alpha amylase microbienne;
(iii) clarifier le substrat avant l'étape de fermentation;
(iv) ajouter des enzymes additionnels à l'étape de mise en contact; et/ou
(v) mettre en contact le substrat avec une solution aqueuse comprenant un contre-courant pour diluer le pourcentage de DS avant l'étape de fermentation.

13. Méthode selon la revendication 11 ou la revendication 12, dans laquelle:
(i) l'alpha-amylase est une alpha-amylase sélectionnée dans le groupe consistant en orge, blé, mélange riz-maïs, seigle, sorgho, riz, millet, triticale, mannioc, pomme de terre, patates, betterave sucrière, canne à sucre, soja et pois; et/ou
(ii) l'amidon granulaire est obtenu de grains, sorgho, orge, blé, riz ou leurs combinaisons.

14. Procédé selon l'une quelconque des revendications 11 à 13, dans lequel l'étape de mise en contact est exécutée à une température entre 50°C et 70°C.

15. procédé selon l'une quelconque des revendications 11 à 14, dans lequel:
(a) les enzymes additionnelles sont sélectionnées dans le groupe de phytases, protéases, cellulases et/ou hémicellulases; ou
(b) l'enzyme additionnelle est une phytase et/ou une protéase; ou
(c) la boue a entre 5-60% DS d'amidon granulaire; ou
(d) le % en DS d'amidon granulaire est entre 20-40% DS.
